# EUROPÄISCHE PATENTSCHRIFT

(11) **EP 0 613 884 B1**
(45) Veröffentlichungstag und Bekanntmachung des Hinweises auf die Patenterteilung: **20.06.2001**
(21) Anmeldenummer: 94102323.6
(22) Anmeldetag: 16.02.1994
(51) Int. Cl.: C07D 207/38, C07D 209/54, C07F 9/572, A01N 43/36, A01N 57/08, A01N 57/24

(54) **Dialkyl-1-H-3-(2,4-dimethylphenyl)-pyrrolidin-2,4-dione, ihre Herstellung und ihre Verwendung als Schädlingsbekämpfungsmittel und Herbizide**
Dialkyl-1-H-3(2,4-dimethylphenyl)-pyrrolidin-2,4-diones, their preparation and their use as parasiticides and herbicides
Dialkyl-1-H-3-(2,4-diméthylphényl)-pyrrolidine-2,4-diones, leur préparation et leur utilisation comme parasiticides et herbicides

(30) Priorität: 01.03.1993 DE 4306259
(43) Veröffentlichungstag der Anmeldung: 07.09.1994
(73) Patentinhaber: BAYER AG, 51368 Leverkusen (DE)
(72) Erfinder: Fischer, Reiner, Dr., D-40789 Monheim (DE); Bretschneider, Thomas, Dr., D-53797 Lohmar (DE); Krüger, Bernd-Wieland, Dr., D-51467 Bergisch Gladbach (DE); Santel, Hans-Joachim, Dr., D-51371 Leverkusen (DE); Dollinger, Markus, Dr., D-51381 Leverkusen (DE); Erdelen, Christoph, Dr., D-42799 Leichlingen (DE); Wachendorff-Neumann, Ulrike Dr., D-40789 Monheim (DE)

(56) Entgegenhaltungen:
- EP-A- 0 456 063
- EP-A- 0 521 334
- EP-A- 0 595 130
- EP-A- 0 596 298

## Beschreibung

Die Erfindung betrifft neue Dialkyl-1-H-3-(2,4-dimethylphenyl)-pyrrolidin-2,4-dione, Verfahren zu ihrer Herstellung und ihre Verwendung als Schädlingsbekämpfungsmittel (insbesondere als Insektizide und Akarizide) und als Herbizide.

Von 3-Acyl-pyrrolidin-2,4-dionen sind pharmazeutische Eigenschaften vorbeschrieben (S. Suzuki et al. Chem. Pharm. Bull. 15 1120 (1967)). Weiterhin wurden N-Phenylpyrrolidin-2,4-dione von R. Schmierer und H. Mildenberger (Liebigs Ann. Chem. 1985 1095) synthetisiert. Eine biologische Wirksamkeit dieser Verbindungen wurde nicht beschrieben.

In EP-A 0 262 399 werden ähnlich strukturierte Verbindungen (3-Aryl-pyrrolidin-2,4-dione) offenbart, von denen jedoch keine herbizide, insektizide oder akarizide Wirkung bekannt geworden ist. Bekannt mit herbizider, insektizider oder akarizider Wirkung sind unsubstituierte, bicyclische 3-Aryl-pyrrolidin-2,4-dion-Derivate (EP-A 355 599 und EP 415 211), substituierte bicyclische 3-Aryl-pyrrolidin-2,4-dion-Derivate (EP 501 129) sowie substituierte mono-cyclische 3-Aryl-pyrrolidin-2,4-dion-Derivate (EP-A 377 893, EP 442 077 und EP 497 127).

Weiterhin bekannt sind polycyclische 3-Arylpyrrolidin-2,4-dion-Derivate (EP 442 073) sowie 1-H-3-Arylpyrrolidin-dion-Derivate (EP 456 063 und EP 521 334).

Es wurden nun neue Dialkyl-1-H-3-(2,4-dimethylphenyl)-pyrrolidin-2,4-dione der Formel (Ic) 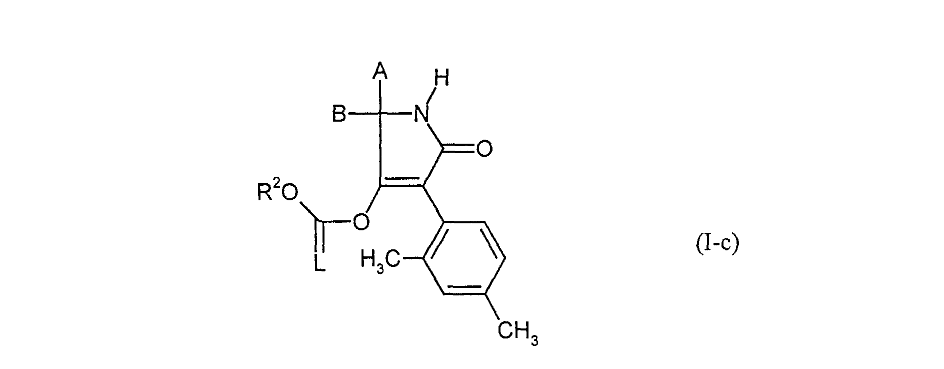 gefunden,
in welcher
- A: für gegebenenfalls durch Halogen-substituiertes geradkettiges oder verzweigtes C₁-C₁₀-Alkyl steht,
- B: für geradkettiges oder verzweigtes C₂-C₁₀-Alkyl steht,
- A, B: und das Kohlenstoffatom an das sie gebunden sind, für einen unsubstituierten C₃-C₁₂-Spirocyclus stehen,
- L: für Sauerstoff oder Schwefel,
- R²: für gegebenenfalls durch Halogen substituiertes C₁-C₂₀-Alkyl, C₃-C₂₀-Alkenyl, C₁-C₈-Alkoxy-C₂-C₈-alkyl, C₁-C₈-Polyalkoxy-C₂-C₈-alkyl steht,
für gegebenenfalls durch Halogen, Nitro, C₁-C₆-Alkyl, C₁-C₆-Alkoxy, C₁-C₆-Halogenalkyl substituiertes Cycloalkyl, Phenyl oder Benzyl steht.

Aufgrund eines oder mehrerer Chiralitätszentren, fallen die Verbindungen der Formel (Ic) im allgemeinen als Stereoisomerengemisch an, die gegebenenfalls in üblicher Art und Weise getrennt werden können. Sie können sowohl in Form ihrer Diastereomerengemische als auch als reine Diastereomere oder Enantiomere verwendet werden. Im folgenden wird der Einfachheit halber stets von Verbindungen der Formel (Ic) gesprochen, obwohl sowohl die reinen Verbindungen, als auch die Gemische mit unterschiedlichen Anteilen an isomeren, enantiomeren und stereomeren Verbindungen gemeint sind.

Weiterhin wurde gefunden, daß man die neuen Dialkyl-1-H-3-(2,4-dimethylphenyl)-pyrrolidin-2,4-dione der Formel (Ic) nach einem der im folgenden beschriebenen Verfahren erhält.

Man erhält Verbindungen der Formel (Ic-1) 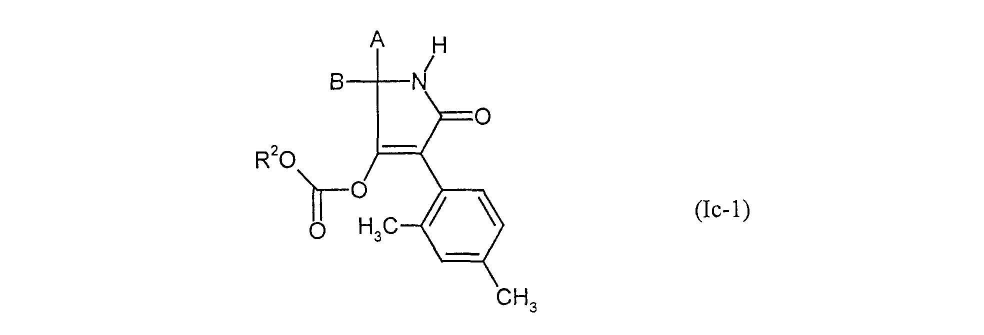 in welcher
A, B und R² die oben angegebene Bedeutung haben,
wenn man Verbindungen der Formel (Ia) 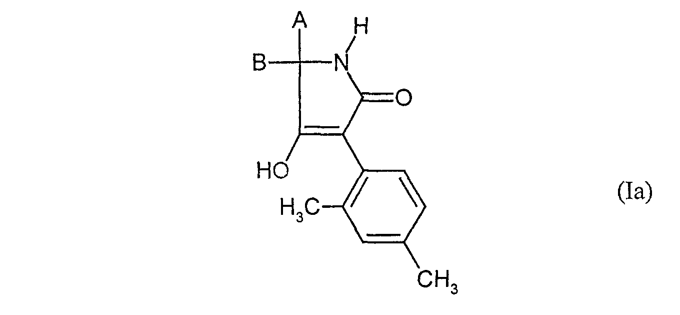 in welcher
A und B die oben angegebene Bedeutung haben,
mit Verbindungen der allgemeinen Formel (V)

R²-O-CO-Cl (V)

in welcher
R² die oben angegebene Bedeutung hat,
gegebenenfalls in Gegenwart eines Verdünnungsmittels und gegebenenfalls in Gegenwart eines Säurebindemittels umsetzt;
oder
(D) man erhält Verbindungen der Formel (Ic-2) 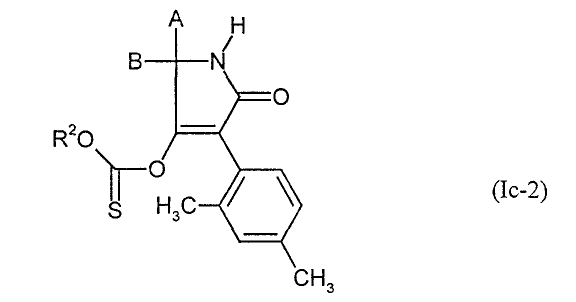 in welcher
A, B und R² die oben angegebene Bedeutung haben,
wenn man Verbindungen der Formel (Ia) 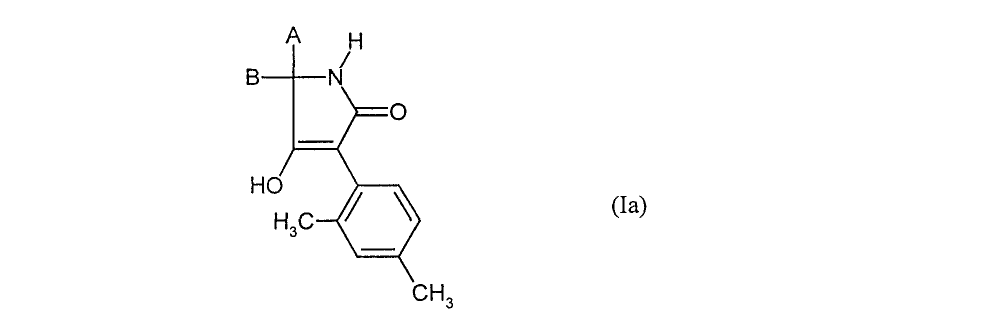 in welcher
A und B die oben angegebene Bedeutung haben,
α) mit Verbindungen der allgemeinen Formel (VI) 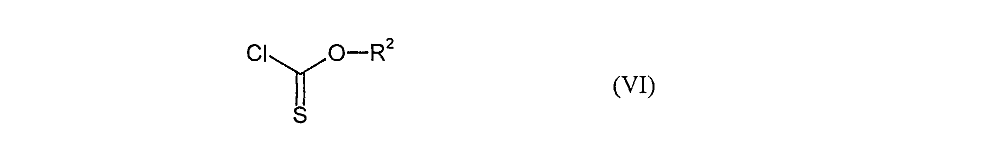 in welcher
   R² die oben angegebene Bedeutung hat,
   gegebenenfalls in Gegenwart eines Verdünnungsmittels und gegebenenfalls in Gegenwart eines Säurebindemittels umsetzt,
   oder
β) mit Schwefelkohlenstoff und anschließend mit Alkylhalogeniden der allgemeinen Formel (VII)

   R²-Hal (VII)

   in welcher
   - R²: die oben angegebene Bedeutung hat
   und
   - Hal: für Chlor, Brom, Iod steht,
   gegebenenfalls in Gegenwart eines Verdünnungsmittels umsetzt.

Weiterhin wurde gefunden, daß sich die neuen Dialkyl-1-H-3-(2,4-dimethylphenyl)-pyrrolidin-2,4-dione der Formel (Ic) durch hervorragende insektizide, akarizide und herbizide Wirkungen auszeichnen.

Für die allgemeinen Formeln der vorliegenden Anmeldung gilt, dass:
- A: bevorzugt für gegebenenfalls durch Fluor und/oder Chlor substituiertes geradkettiges oder verzweigtes C₁-C₈-Alkyl steht,
- B: bevorzugt für geradkettiges oder verzweigtes C₂-C₈-Alkyl steht oder
- A,B: und das Kohlenstoffatom an das sie gebunden sind bevorzugt für einen unsubstituierten C₃-C₈-Spirocyclus stehen,
- A: besonders bevorzugt für gegebenenfalls durch Fluor substituiertes geradkettiges oder verzweigtes C₁-C₆-Alkyl steht,
- B: besonders bevorzugt für geradkettiges oder verzweigtes C₂-C₄-Alkyl steht oder
- A, B: und das Kohlenstoffatom an das sie gebunden sind ganz besonders bevorzugt für einen unsubstituierten C₃-C₇-Spirocyclus stehen,
- L: für Sauerstoff oder Schwefel steht,
- R²: bevorzugt für gegebenenfalls durch Halogen substituiertes C₁-C₁₆-Alkyl, C₃-C₁₆-Alkenyl, C₁-C₆-Alkoxy-C₂-C₆-alkyl, C₁-C₆-Polyalkoxy-C₂-C₆-alkyl steht,
für gegebenenfalls durch Halogen, Nitro, C₁-C₄-Alkyl, C₁-C₃-Alkoxy, C₁-C₃-Halogenalkyl substituiertes Cycloalkyl, Phenyl oder Benzyl steht,
- R²: besonders bevorzugt für gegebenenfalls durch Fluor oder Chlor substituiertes C₁-C₁₄-Alkyl, C₃-C₁₄-Alkenyl, C₁-C₄-Alkoxy-C₂-C₆-alkyl, C₁-C₄-Polyalkoxy-C₂-C₆-alkyl steht,
oder für gegebenenfalls durch Fluor, Chlor, Nitro, Methyl, Ethyl, Propyl, i-Propyl, Methoxy, Ethoxy, Trifluormethyl substituiertes Cycloalkyl, Phenyl oder Benzyl steht,

Verwendet man gemäß Verfahren (C) 3-(2,4-Dimethylphenyl)-5-sec.-butyl-5-methyl-pyrrolidin-2,4-dion und Chlorameisensäureethoxyethylester als Ausgangsverbindungen, so kann der Verlauf des erfindungsgemäßen Verfahrens durch folgendes Reaktionsschema wiedergegeben werden:

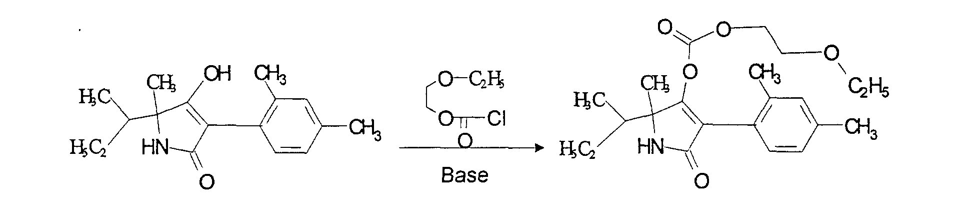

Verwendet man gemäß Verfahren (D_{α}) 3-(2,4-Dimethylphenyl)-5-isopropyl-5-methyl-pyrrolidin-2,4-dion und Chlormonothioameisensäuremethylester als Ausgangsprodukte, so kann der Reaktionsverlauf wie folgt wiedergegeben werden:

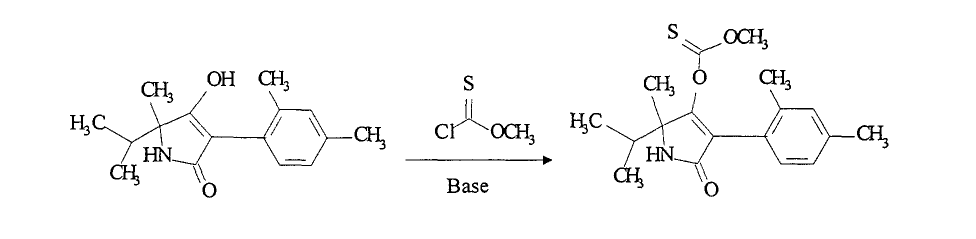

Im einzelnen seien außer den bei den Herstellungsbeispielen genannten Verbindungen die folgenden Verbindungen der Formel (Ic) genannt:

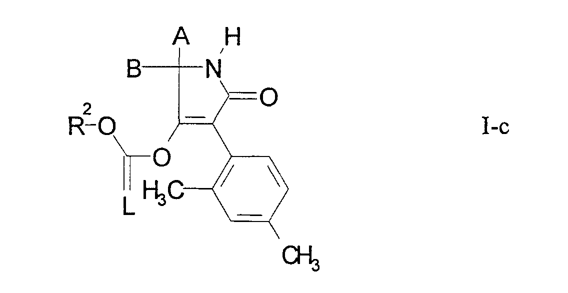

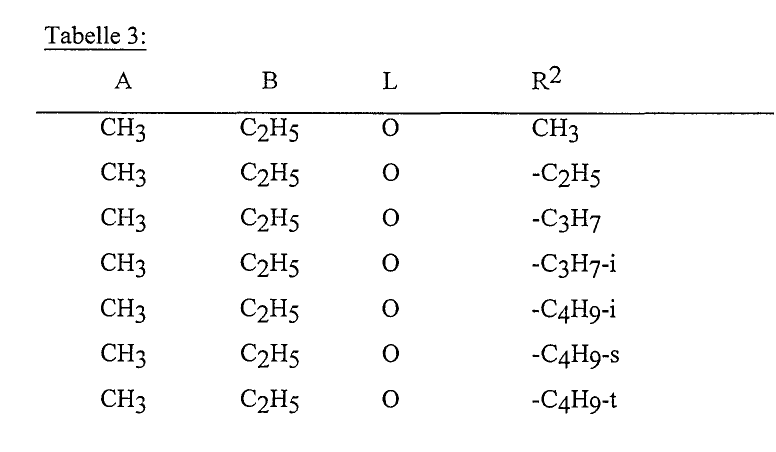

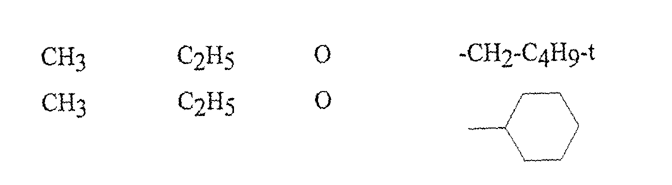

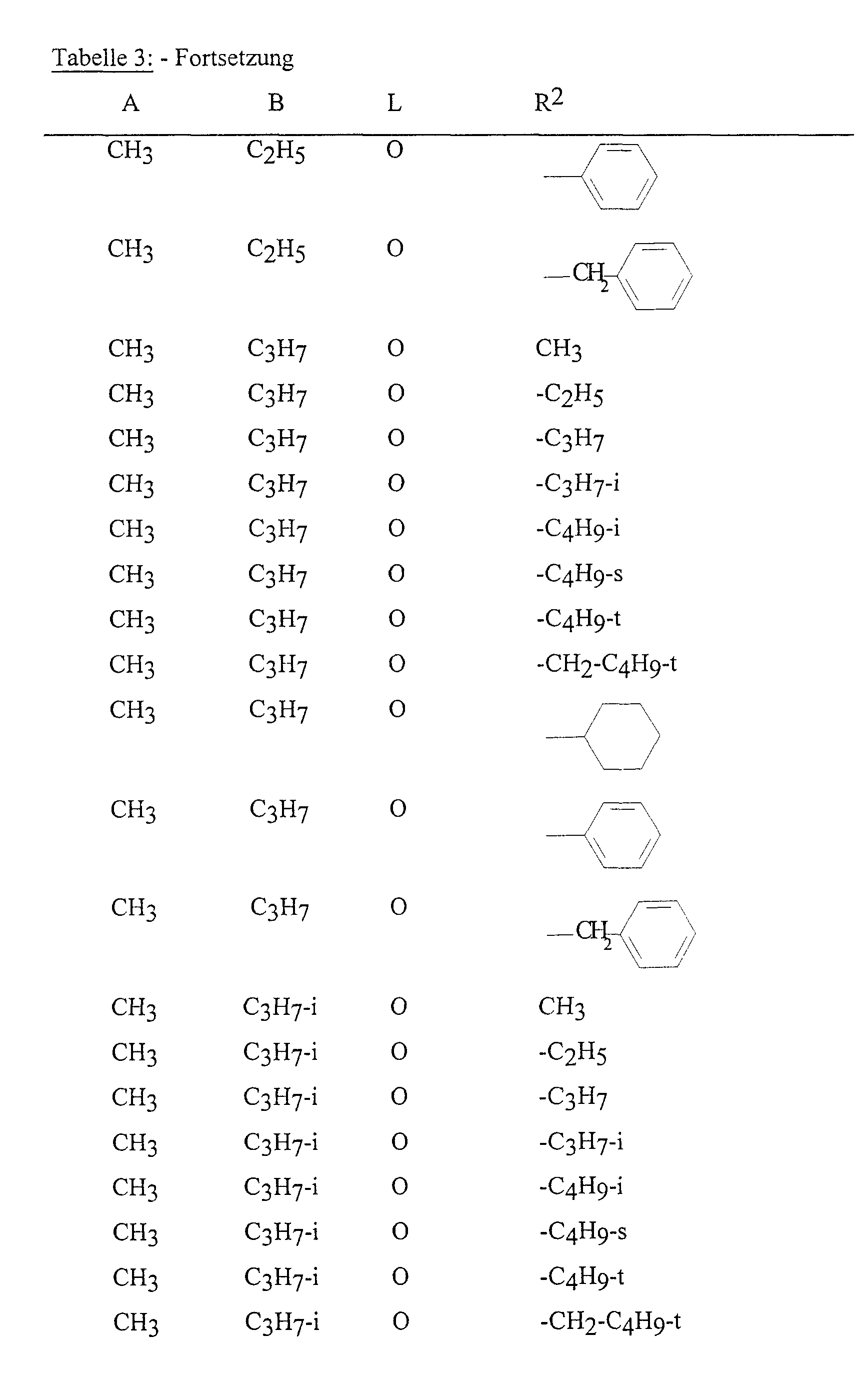

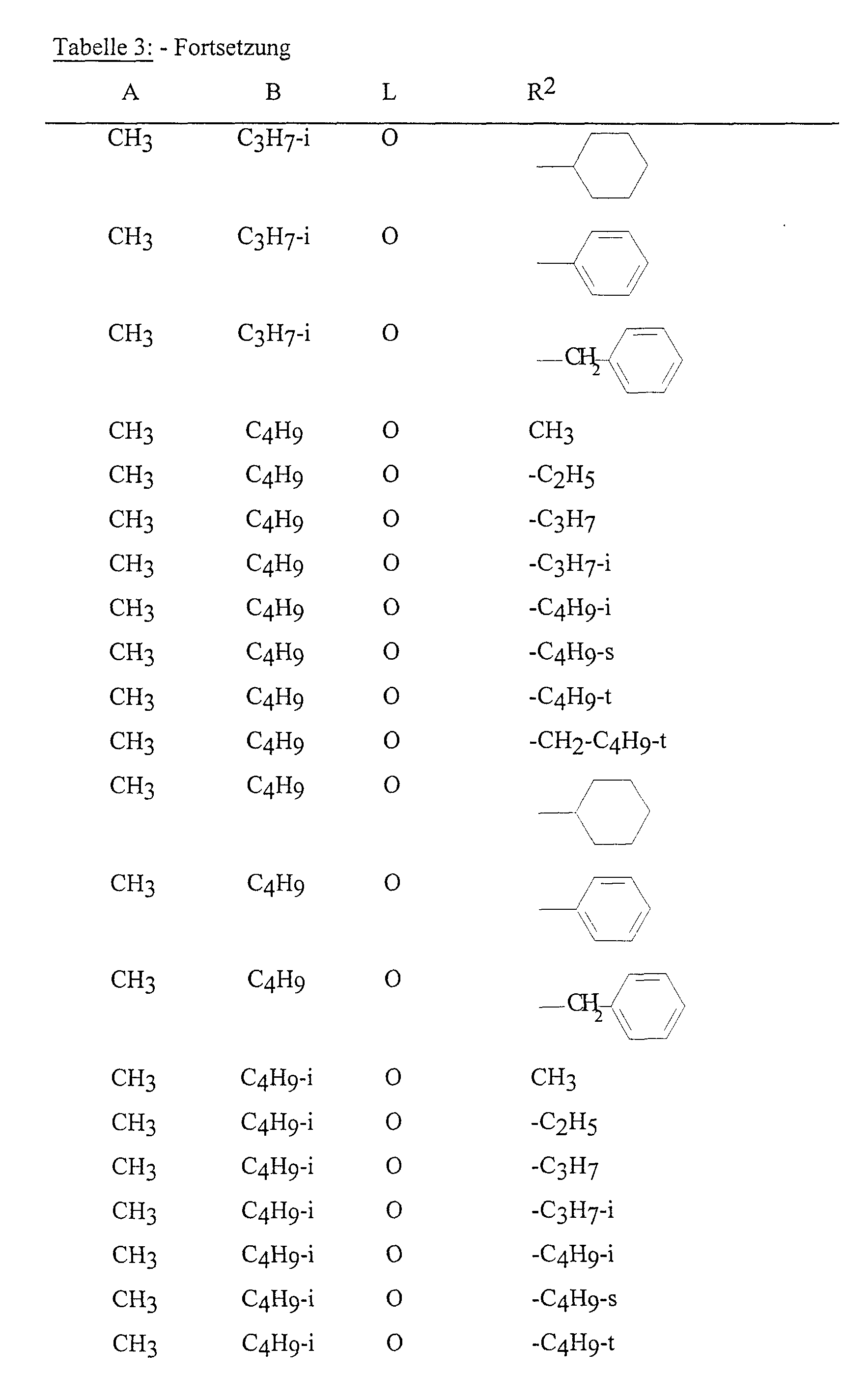

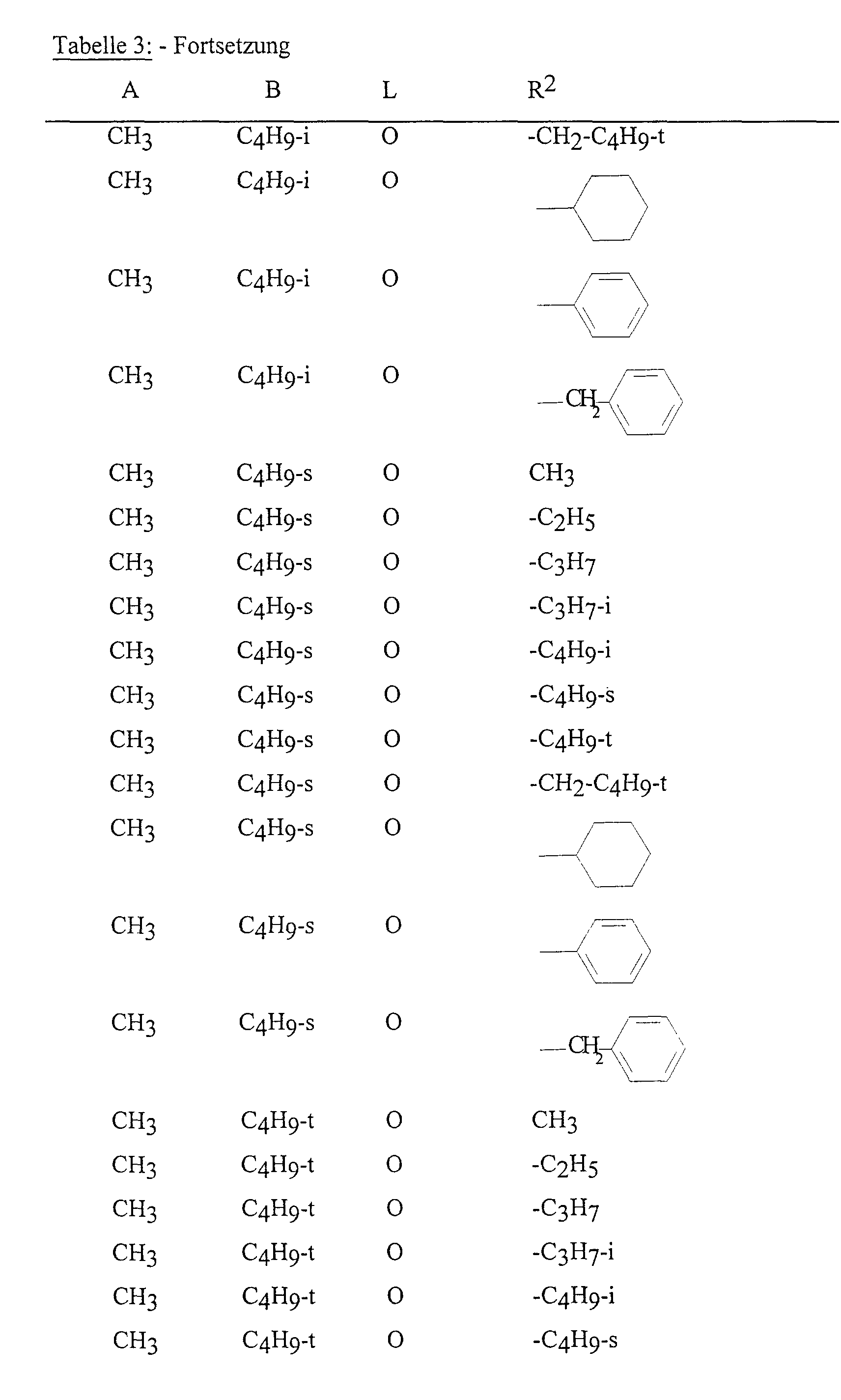

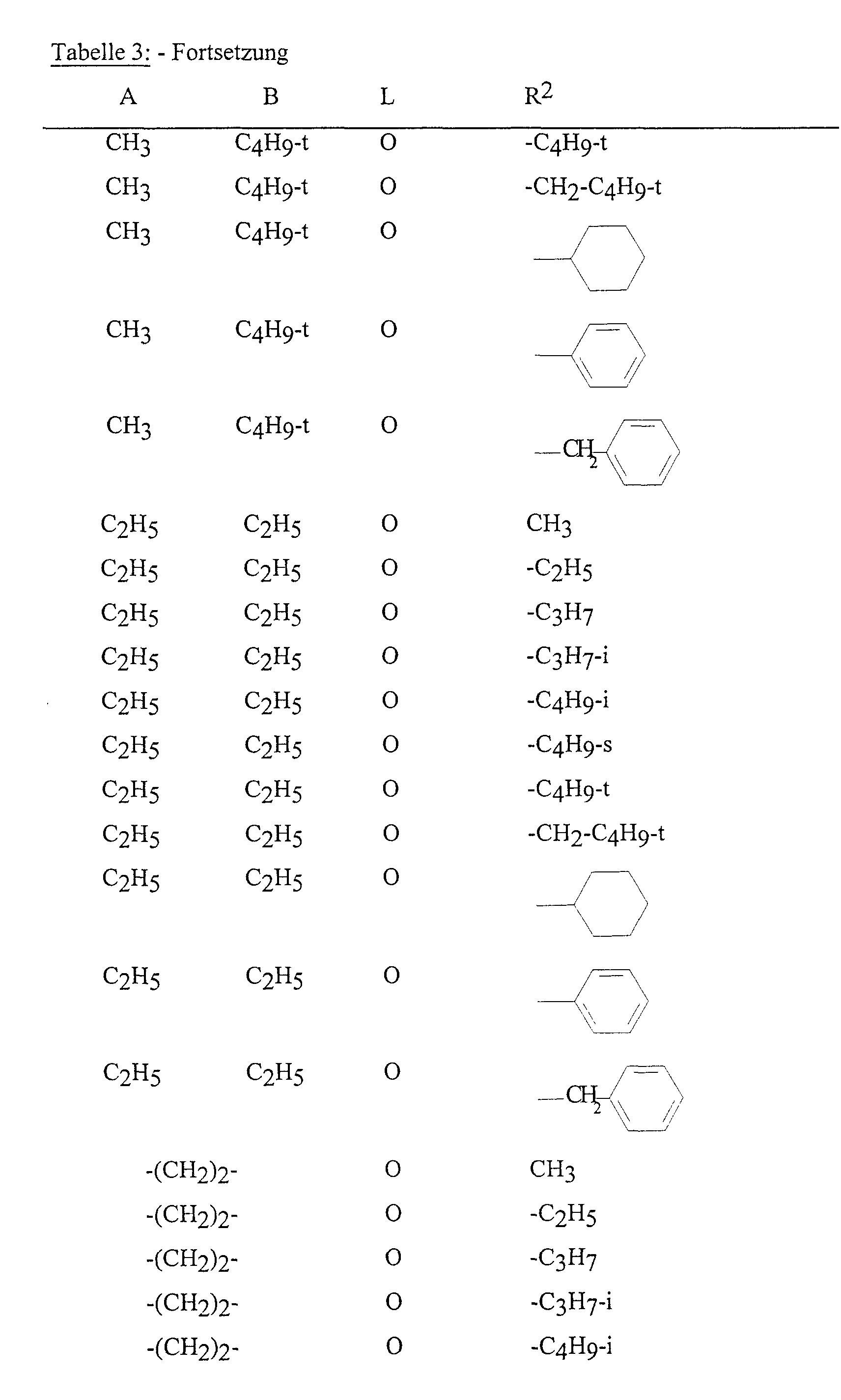

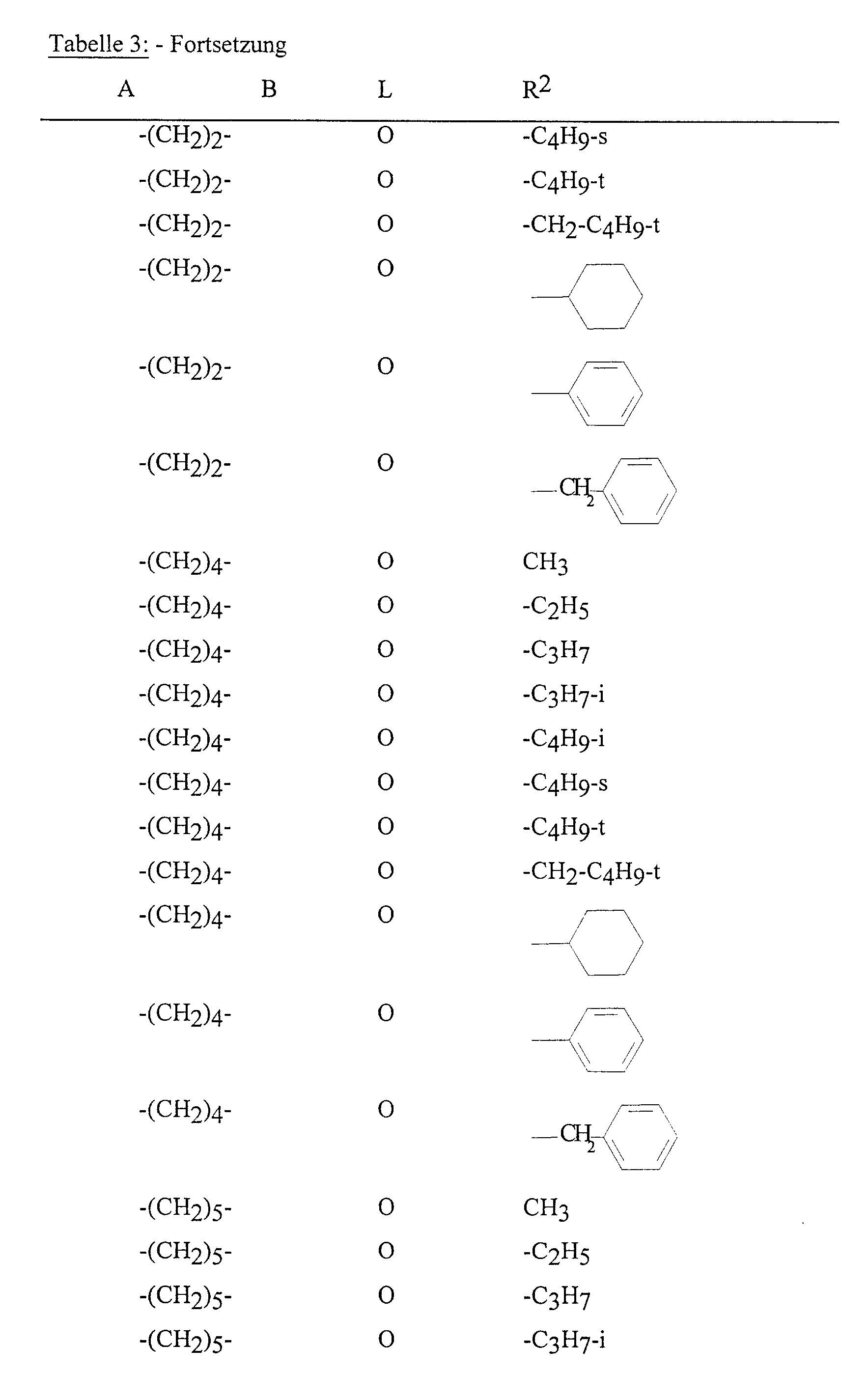

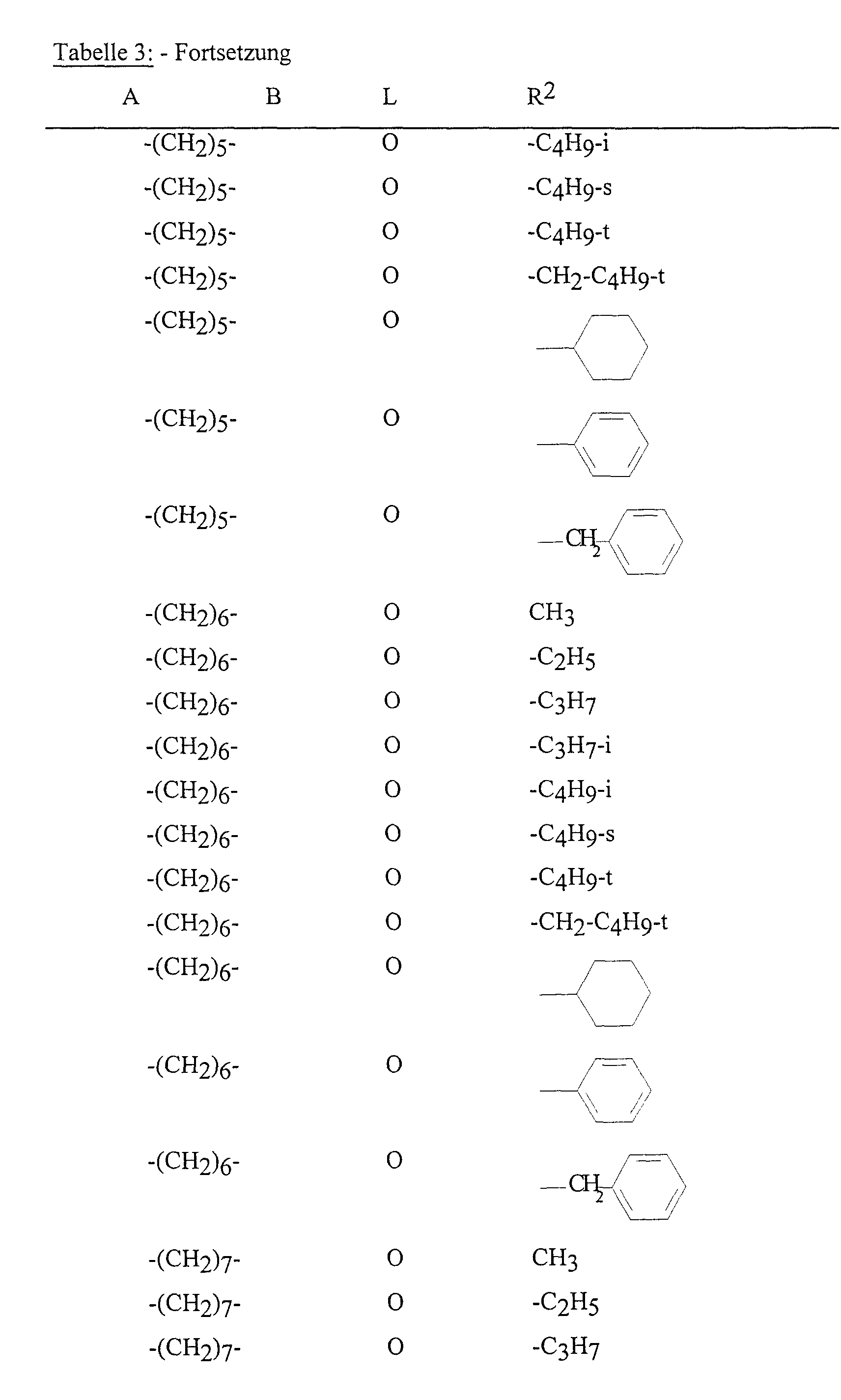

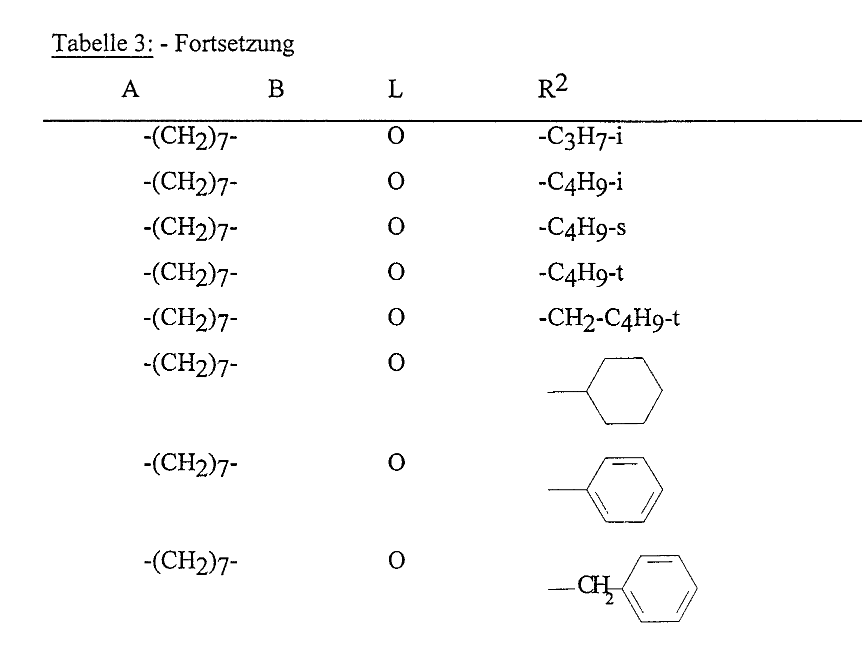

Die zur Durchführung der erfindungsgemäßen Verfahren (C) und (D) als Ausgangsstoffe benötigten Verbindungen der Formel (V) und (VI) sind allgemein bekannte Verbindungen der organischen bzw. anorganischen Chemie.

Das Verfahren (C) ist dadurch gekennzeichnet, daß man Verbindungen der Formel (Ia) mit Verbindungen der Formel (V) umsetzt.

Verwendet man die entsprechenden Verbindungen der Formel (V) so kommen als Säurebindemittel bei der Umsetzung nach dem erfindungsgemäßen Verfahren (C) alle üblichen Säureakzeptoren in Betracht. Vorzugsweise verwendbar sind tertiäre Amine, wie Triethylamin, Pyridin, DABCO, DBU, DBA, Hünig-Base und N,N-Dimethyl-anilin, ferner Erdalkalimetalloxide, wie Magnesium- und Calciumoxid, außerdem Alkali- und Erdalkalimetallcarbonate, wie Natriumcarbonat, Kaliumcarbonat und Calciumcarbonat sowie Alkalihydroxide wie Natriumhydroxid und Kaliumhydroxid.

Als Verdünnungsmittel können bei dem erfindungsgemäßen Verfahren (C) bei Verwendung der Verbindungen der Formel (V) alle gegenüber diesen Verbindungen inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenstoff, Chlorbenzol und o-Dichlorbenzol, außerdem Ketone, wie Aceton und Methylisopropylketon, weiterhin Ether, wie Diethylether, Tetrahydrofuran und Dioxan, darüberhinaus Carbonsäureester, wie Ethylacetat, und auch stark polare Solventien, wie Dimethylsulfoxid und Sulfolan.

Bei Verwendung der Verbindungen als Carbonsäure-Derivate der Formel (V) können die Reaktionstemperaturen bei der Durchführung des erfindungsgemäßen Verfahrens (C) innerhalb eines größeren Bereiches variiert werden. Arbeitet man in Gegenwart eines Verdünnungsmittels und eines Säurebindemittels, so liegen die Reaktionstemperaturen im allgemeinen zwischen -20°C und +100°C, vorzugsweise zwischen 0°C und 50°C.

Das erfindungsgemäße Verfahren (C) wird im allgemeinen unter Normaldruck durchgeführt.

Bei der Durchführung des erfindungsgemäßen Verfahrens (C) werden die Ausgangsstoffe der Formel (Ia) und die entsprechenden Verbindungen der Formel (V) im allgemeinen in angenähert äquivalenten Mengen verwendet. Es ist jedoch auch möglich, die eine oder andere Komponente in einem größeren Überschuß (bis zu 2 Mol) einzusetzen. Die Aufarbeitung erfolgt dann nach üblichen Methoden. Im allgemeinen geht man so vor, daß man ausgefallene Salze entfernt und das verbleibende Reaktionsgemisch durch Abziehen des Verdünnungsmittels einengt.

Beim Herstellungsverfahren (D) setzt man pro Mol Ausgangsverbindung der Formel (Ia) ca. 1 Mol der Verbindung der Formel (VI) bei 0 bis 120°C, vorzugsweise bei 20 bis 60°C um.

Als gegebenenfalls zugesetzte Verdünnungsmittel kommen alle inerten polaren organischen Lösungsmittel in Frage, wie Ether, Amide, Sulfone, Sulfoxide.

Vorzugsweise werden Dimethylsulfoxid, Tetrahydrofuran, Dimethylformamid, Dimethylsulfid eingesetzt.

Stellt man in einer bevorzugten Ausführungsform durch Zusatz von starken Deprotonierungsmitteln wie z.B. Natriumhydrid oder Kaliumtertiärbutylat das Enolatsalz der Verbindung (Ia) dar, kann auf den weiteren Zusatz von Säurebindemitteln verzichtet werden.

Werden Säurebindemittel eingesetzt, so kommen übliche anorganische oder organische Basen in Frage, beispielhaft seien Natriumhydroxid, Natriumcarbonat, Kaliumcarbonat, Pyridin, Triethylamin aufgeführt.

Die Reaktion kann bei Normaldruck oder unter erhöhtem Druck durchgeführt werden, vorzugsweise wird bei Normaldruck gearbeitet. Die Aufarbeitung geschieht nach üblichen Methoden.

Die Wirkstoffe eignen sich zur Bekämpfung von tierischen Schädlingen, vorzugsweise Arthropoden und Nematoden, insbesondere Insekten und Spinnentieren, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.

Aus der Ordnung der Diplopoda z.B. Blaniulus guttulatus
Aus der Ordnung der Chilopoda z.B. Geophilus carpophagus, Scutigera spec.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.

Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.
Aus der Ordnung der Dermaptera z.B. Forficula auricularia.
Aus der Ordnung der Isoptera z.B. Reticulitermes spp..

Aus der Ordnung der Anoplura z.B. Phylloxera vastatrix, Pemphigus spp., Pediculus humanus corporis, Haematopinus spp., Linognathus spp..
Aus der Ordnung der Mallophaga z.B. Trichodectes spp., Damalinea spp.
Aus der Ordnung der Thysanoptera z.B. Hercinothrips femoralis, Thrips tabaci.
Aus der Ordnung der Heteroptera z.B. Eurygaster spp., Dysdercus intermedius,
Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Spodoptera exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp..
Aus der Ordnung der Arachnida z.B. Scorpio maurus, Latrodectus mactans.

Aus der Ordnung der Acarina z.B. Acarus siro, Argas spp., Omithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Die erfindungsgemäßen Wirkstoffe wirken nicht nur gegen Pflanzen-, Hygiene- und Vorratsschädlinge, sondern auch auf dem veterinärmedizinischen Sektor gegen tierische Parasiten (Ektoparasiten und Endoparasiten) wie Schildzecken, Lederzecken, Räudemilben, Laufmilben, Fliegen (stechend und leckend), parasitierende Fliegenlarven, Läuse, Haarlinge, Federlinge, Flöhe und endoparasitisch lebende Würmer.

Sie sind gegen normalsensible und resistente Arten und Stämme sowie gegen alle parasitierenden und nicht parasitierenden Entwicklungsstadien der Ekto- und Endoparasiten wirksam.

Die erfindungsgemäßen Wirkstoffe zeichnen sich durch eine hohe insektizide und akarizide Wirksamkeit aus.

Sie lassen sich mit besonders gutem Erfolg zur Bekämpfung von pflanzenschädigenden Insekten, wie beispielsweise gegen die Larven der grünen Reiszikade (Nephotettix cincticeps), gegen die Larven des Meerettichblattkäfers (Phaedon cochleariae) oder gegen die Tabakknospenraupe (Heliothis virescens) einsetzen.

Darüberhinaus lassen sie sich hervorragend zur Bekämpfung von pflanzenschädigenden Milben, wie beispielsweise gegen die gemeine Spinnmilbe (Tetranychus urticae) und gegen die Obstbaumspinnmilbe (Panonychus ulmi), einsetzen.

Die erfindungsgemäßen Wirkstoffe können weiterhin als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Die erfindungsgemäßen Wirkstoffe eignen sich sehr gut zur selektiven Bekämpfung monokotyler Unkräuter in monokotylen und dikotylen Kulturen im Nachauflaufverfahren. Sie können beispielsweise in Soja oder Winterweizen mit sehr gutem Erfolg zur Bekämpfung von Schadgräsern eingesetzt werden.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Einweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarb-stoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion (AMETHYDIONE) oder N-(2-Benzthiazolyl)-N,N'-dimethyl-hamstoff (METABENZTHIAZURON) zur Unkrautbekämpfung in Getreide; 4-Amino-3-methyl-6-phenyl-1,2,4-triazin-5(4H)-on (METAMITRON) zur Unkrautbekämpfung in Zuckerrüben und 4-Amino-6-(1,1-dimethylethyl)-3-methylthio-1,2,4-triazin-5(4H)-on (METRIBUZIN) zur Unkrautbekämpfung in Sojabohnen, in Frage. Weiterhin kommen 2,4-Dichlorphenoxyessigsäure (2,4-D); 4-(2,4-Dichlorphenoxy)-buttersäure (2,4-DB); 2,4-Dichlorphenoxypropionsäure (2,4-DP); 3-Isopropyl-2,1,3-benzothiadiazin-4-on-2,2-dioxid (BENTAZON); Methyl-5-(2,4-dichlorphenoxy)-2-nitrobenzoat (BIFENOX); 3,5-Dibrom-4-hydroxy-benzonitril (BROMOXYNIL); 2-Chlor-N-{[(4-methoxy-6-methyl-1,3,5-triazin-2-yl)-amino]-carbonyl}-benzolsulfonamid (CHLORSULFURON); 2-[4-(2,4-Dichlorphenoxy)-phenoxy]-propionsäure, deren Methyl- oder deren Ethylester (DICLOFOPMETHYL); 4-Amino-6-t-butyl-3-ethylthio-1,2,4-triazin-5(4H)-on (ETHIOZIN); 2-(4-[(6-Chlor-2-benzoxazolyl)-oxy]-phenoxy}-propansäure, deren Methyl- oder deren Ethylester (FENOXAPROP); [(4-Amino-3,5-dichlor-6-fluor-2-pyridinyl)-oxy]-essigsäure bzw. deren 1-Methylheptylester (FLUROXYPYR); Methyl-2-[4,5-dihydro-4-methyl-4-(1-methylethyl)-5-oxo-1H-imidazol-2-yl]-4(5)-methylbenzoat (IMAZAMETHABENZ); 3,5-Diiod-4-hydroxybenzonitril (IOXYNIL); N,N-Dimethyl-N'-(4-isopropylphenyl)-hamstoff (ISOPROTURON); (2-Methyl-4-chlorphenoxy)-essigsäure (MCPA); (4-Chlor-2-methylphenoxy)-propionsäure (MCPP); N-Methyl-2-(1,3-benzthiazol-2-yloxy)-acetanilid (MEFENACET); 2-{[[((4-Methoxy-6-methyl-1,3,5-triazin-2-yl)-amino)-carbonyl]-amino]-sulfonyl}-benzoesäure oder deren (METSULFURON); N-(1-Ethylpropyl)-3,4-dimethyl-2,6-dinitroanilin (PENDIMETHALIN); 0-(6-Chlor-3-phenylpyridazin-4-yl)-S-octylthiocarbonat (PYRIDATE); 4-Ethylamino-2-t-butylamino-6-methylthio-s-triazin (TERBUTRYNE); 3-[[[[(4-Methoxy-6-methyl-1,3,5-triazin-2-yl)-amino]-carbonyl]-amino]-sulfonyl]-thiophen-2-carbonsäure (THIAMETURON) in Frage. Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

Die Herstellung und die Verwendung der erfindungsgemäßen Wirkstoffe geht aus den nachfolgenden Beispielen hervor.

### Herstellungsbeispiele

### Beispiel (Ic-1)

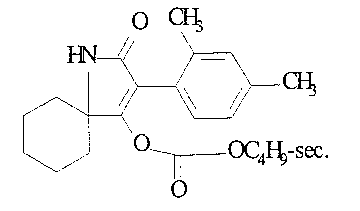

5,42 g (0,020 Mol) 3-(2,4-Dimethylphenyl)-5,5-pentamethylen-pyrrolidin-2,4-dion werden in 70 ml absolutem Dichlormethan vorgelegt und mit 2,8 ml Triethylamin versetzt. Bei 0-10°C werden 2,73 g Chlorameisensäure-sec.-butylester in 5 ml absolutem Dichlormethan zugegeben und der Ansatz bei Raumtemperatur weitergerührt. Das Ende der Reaktion wird dünnschichtchromatographisch ermittelt. Anschließend wird zweimal mit jeweils 200 ml 0,5N Natronlauge gewaschen, die organische Phase über Magnesiumsulfat getrocknet und das Lösungsmittel im Vakuum abgezogen.

Man erhält 1,97 g (26 % der Theorie) Kohlensäure-O-(sec.-butylester-O-[3-(2,4-dimethylphenyl)]-5,5-pentamethylen-Δ3-pyrrolin-4-yl-2-on vom Schmelzpunkt Fp.: 169°C.

Analog zu Beispiel (Ic-1) und gemäß den allgemeinen Angaben in der Beschreibung zu den erfindungsgemäßen Verfahren werden die nachfolgend in Tabelle 9 aufgeführten Endprodukte der Formel (Ic) erhalten

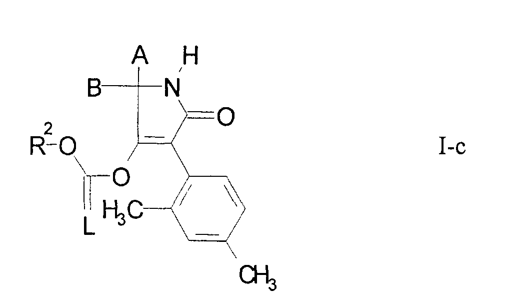

**Tabelle 9:**

| Bsp.-Nr. | A | B | L | R² | physikal. Konst. [°C] |
|---|---|---|---|---|---|
| Ic-2 | CH₃ | i-C₃H₇ | O | C₂H₅ | Fp.: 149 |
| Ic-3 | CH₃ | i-C₃H₇ | O | s-C₄H₉ | Fp.: 132 |
| Ic-4 | -(CH₂)₅- | | O | C₂H₅ | Fp.: 181 |
| Ic-6 | CH₃ | i-C₃H₇ | O | CH₃ | Fp.: 152 |
| 1c-7 | CH3 | i-C₃H₇ | O | i-C₃H₇ | Öl |
| Ic-8 | CH₃ | i-C₃H₇ | O | s-C₄H₉ | Fp.: 36 |
| Ic-9 | CH₃ | s-C₄H₉ | O | C₂H₅ | Fp.: 60 |
| Ic-10 | CH₃ | s-C₄H₉ | O | s-C₄H₉ | Öl |

### Anwendungsbeispiele:

In den folgenden Anwendungsbeispielen wurden die nachstehend aufgeführten Verbindungen als Vergleichssubstanzen eingesetzt: 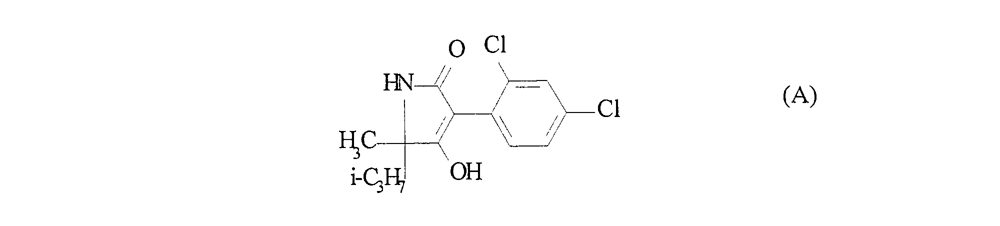 3-(2,4-Dichlorphenyl)-5-methyl-5-isopropyl-pyrrolidin-2,4-dion bekannt aus EP 456 063 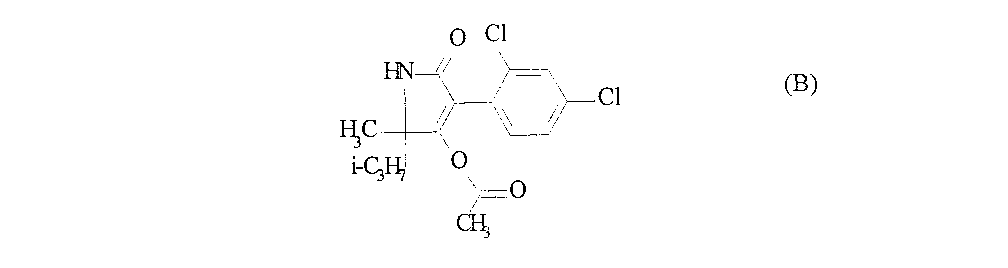 3-(2,4-Dichlorphenyl)-5-methyl-5-isopropyl-4-acetoxy-A3-pyrrolin-2-on, bekannt aus EP 456 063 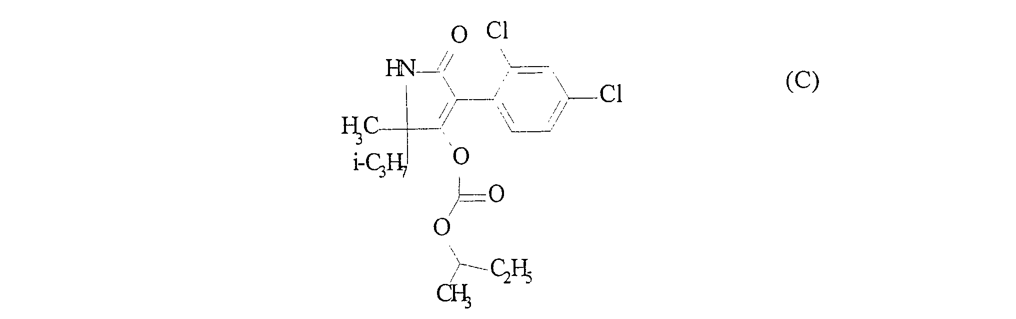 Kohlensäure-O-(sec.-butylester-O-[3-(2,4-dichlorphenyl)]-5-methyl-5-isopropyl-Δ3-pyrrolin-4-yl-2-on, bekannt aus EP 456 063 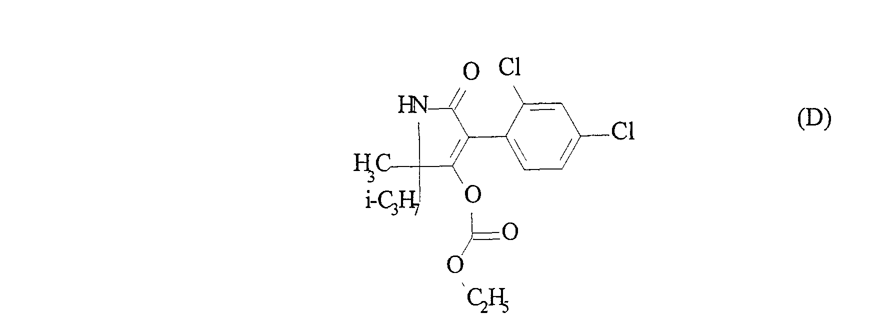 Kohlensäure-O-(ethylester-O-[3 -(2,4-dichlorphenyl)]-5-methyl-5-isopropyl-Δ3-pyrrolin-4-yl-2-on bekannt aus EP 456 063 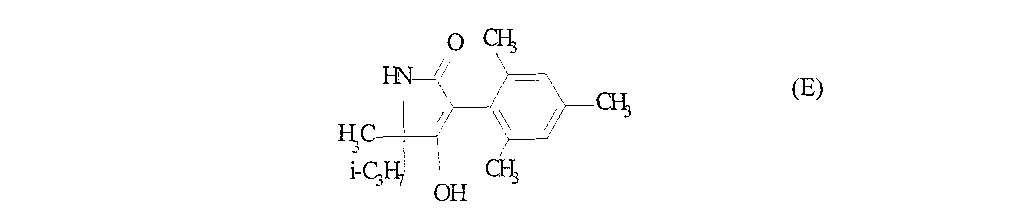 3-(2,4,6-Trimethylphenyl)-5-methyl-5-isopropyl-pyrrolidin-2,4-dion, bekannt aus EP 456 063

### Beispiel A

### Post-emergence-Test

- Lösungsmittel:: 5 Gewichtsteile Aceton
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 1 Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.

Es bedeuten:
0 % = keine Wirkung (wie unbehandelte Kontrolle)
100 % = totale Vernichtung

Bei diesem Test wurden mit einer beispielhaften Aufwandmenge von 125 g/ha bei einer guten bis sehr guten Verträglichkeit durch Soja die folgenden Ergebnisse erhalten:

| Pflanze | % Wirkung | Verbindung des Herstellungsbeispiels Nr. |
|---|---|---|
| Digitaria | > 70 | Ic-2, Ic-3 |
| Cynodon | > 40 | Ic-2, Ic-3 |
| Setaria | 90 | Ic-2, Ic-3 |

### Beispiel B

### Phaedon-Larven-Test

- Lösungsmittel:: 7 Gewichtsteile Dimethylformamid
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Meerrettichblattkäfer-Larven (Phaedon cochleariae) besetzt, solange die Blätter noch feucht sind.

Nach der gewünschten Zeit werden die Pflanzen mit Meerrettichblattkäfer-Larven (Phaedon cochleariae) besetzt. Nach jeweils 7 Tagen wird die Abtötung in % bestimmt. Dabei bedeutet 100 %, daß alle Käfer-Larven abgetötet wurden; 0 % bedeutet, daß keine Käfer-Larven abgetötet wurden.

Bei diesem Test bewirkten z.B. die Verbindungen gemäß der Herstellungsbeispiele (Ic-2) bei einer beispielhaften Wirkstoffkonzentration von 0.01 % eine Abtötung von 100 % nach 7 Tagen während die bekannten Verbindungen (A), (B) und (C) keine Abtötung bewirkten.

### Beispiel C

### Heliothis virescens- Test

- Lösungsmittel:: 7 Gewichtsteile Dimethylformamid
- Emulgator :: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Sojatriebe (Glycine max.) werden durch Tauchen in die Wirkstoffzubereitung der gewünschten Konzentration behandelt und mit der Tabakknospenraupe (Heliothis virescens) besetzt, solange die Blätter noch feucht sind.

Nach der gewünschten Zeit wird die Abtötung in % bestimmt. Dabei bedeutet 100 %, daß alle Tiere abgetötet wurden; 0 % bedeutet, daß keine Raupen abgetötet wurden.

Bei diesem Test bewirkten z.B. die Verbindungen der Herstellungsbeispiele überlegene Wirkung gegenüber dem Stand der Technik: (Ic-1), (Ic-3) und (Ic-4) bei einer beispielhaften Wirkstoffkonzentration von 0.1 % eine Abtötung von 100 % nach 7 Tagen, während die aus dem Stand der Technik bekannten Verbindungen (A), (C) und (D) keine Abtötung bewirkten.

### Beispiel E

### Tetranychus-Test (OP-resistent)

- Lösungsmittel:: 3 Gewichtsteile Dimethylformamid
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Bohnenpflanzen (Phaseolus vulgaris), die stark von allen Entwicklungsstadien der gemeinen Spinnmilbe oder Bohnenspinnmilbe (Tetranychus urticae) befallen sind, werden durch Tauchen in die Wirkstoffzubereitung der gewünschten Konzentration behandelt.

Nach der gewünschten Zeit wird die Abtötung in % bestimmt. Dabei bedeutet 100 %, daß alle Spinnmilben abgetötet wurden; 0 % bedeutet, daß keine Spinnmilben abgetötet wurden.

Bei diesem Test bewirkten z.B. die Verbindungen gemäß den Herstellungsbeispielen (Ic-1), (Ic-2), (Ic-3) und (Ic-4) bei einer beispielhaften Wirkstoffkonzentration von 0.02 % eine Abtötung von 100 %, nach 14 Tagen.

### Beispiel F

### Panonychus-Test

- Lösungsmittel:: 3 Gewichtsteile Dimethylformamid
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Ca. 30 cm hohe Pflaumenbäumchen (Prunus domestica), die stark von allen Entwicklungsstadien der Obstbaumspinnmilbe (Panonychus ulmi) befallen sind, werden durch Tauchen in die Wirkstoffzubereitung der gewünschten Konzentration behandelt.

Nach der gewünschten Zeit wird die Abtötung in % bestimmt. Dabei bedeutet 100 %, daß alle Spinnmilben abgetötet wurden; 0 % bedeutet, daß keine Spinnmilben abgetötet wurden.

Bei diesem Test bewirkten z.B. die Verbindungen gemäß den Herstellungsbeispielen (Ic-2) und (Ic-3) bei einer beispielhaften Wirkstoffkonzentration von 0.02 % eine Abtötung von 100 % nach 14 Tagen.

## Patentansprüche

1. Dialkyl-1-H-3-(2,4-dimethylphenyl)-pyrrolidin-2,4-dione der Formel (I-c) 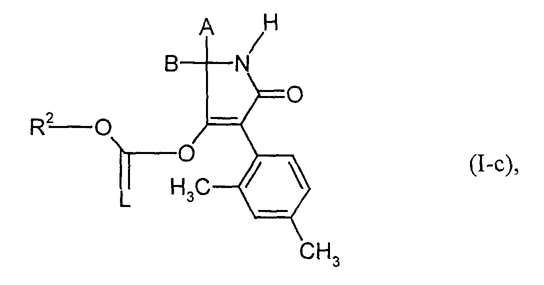 in welcher
A für gegebenenfalls durch Halogen-substituiertes geradkettiges oder verzweigtes C₁-C₁₀-Alkyl steht,
B für geradkettiges oder verzweigtes C₂-C₁₀-Alkyl steht,
A, B und das Kohlenstoffatom an das sie gebunden sind, bevorzugt für einen unsubstituierten C₃-C₁₂-Spirocyclus stehen,
L für Sauerstoff oder Schwefel,
R² für gegebenenfalls durch Halogen substituiertes C₁-C₂₀-Alkyl, C₃-C₂₀-Alkenyl, C₁-C₈-Alkoxy-C₂-C₈-alkyl, C₁-C₈-Polyalkoxy-C₂-C₈-alkyl steht,
für gegebenenfalls durch Halogen, Nitro, C₁-C₆-Alkyl, C₁-C₆-Alkoxy, C₁-C₆-Halogenalkyl substituiertes Cycloalkyl, Phenyl oder Benzyl steht.

2. Dialkyl-1-H-3-(2,4-dimethylphenyl)-pyrrolidin-2,4-dione der Formel (I-c) gemäß Anspruch 1, in welcher
A für gegebenenfalls durch Fluor und/oder Chlor substituiertes geradkettiges oder verzweigtes C₁-C₈-Alkyl steht,
B für geradkettiges oder verzweigtes C₂-C₈-Alkyl steht
oder
A, B und das Kohlenstoffatom, an das sie gebunden sind, für einen unsubstituierten C₃-C₈-Spirocyclus stehen,
L für Sauerstoff oder Schwefel steht,
R² für gegebenenfalls durch Halogen substituiertes C₁-C₁₆-Alkyl, C₃-C₁₆-Alkenyl, C₁-C₆-Alkoxy-C₂-C₆-alkyl, C₁-C₆-Polyalkoxy-C₂-C₆-alkyl steht,
für gegebenenfalls durch Halogen, Nitro, C₁-C₄-Alkyl, C₁-C₃-Alkoxy, C₁-C₃-Halogenalkyl substituiertes Cycloalkyl, Phenyl oder Benzyl steht.

3. Dialkyl-1-H-3-(2,4-dimethylphenyl)-pyrrolidin-2,4-dione der Formel (I-c) gemäß Anspruch 1, in welcher
A für gegebenenfalls durch Fluor substituiertes geradkettiges oder verzweigtes C₁-C₆-Alkyl steht,
B für geradkettiges oder verzweigtes C₂-C₄-Alkyl steht
oder
A, B und das Kohlenstoffatom, an das sie gebunden sind, für einen unsubstituierten C₃-C₇-Spirocyclus stehen,
L für Sauerstoff oder Schwefel,
R² für gegebenenfalls durch Fluor oder Chlor substituiertes C₁-C₁₄-Alkyl, C₃-C₁₄-Alkenyl, C₁-C₄-Alkoxy-C₂-C₆-alkyl, C₁-C₄-Polyalkoxy-C₂-C₆-alkyl steht,
für gegebenenfalls durch Fluor, Chlor, Nitro, Methyl, Ethyl, Propyl, i-Propyl, Methoxy, Ethoxy, Trifluormethyl substituiertes Cycloalkyl, Phenyl oder Benzyl steht.

4. Verfahren zur Herstellung der Dialkyl-1-H-3-(2,4-dimethylphenyl)-pyrrolidin-2,4-dione der Formel (I-c) gemäß Anspruch 1, dadurch gekennzeichnet, daß
C) Für den Fall von Verbindungen der Formel (Ic-1) 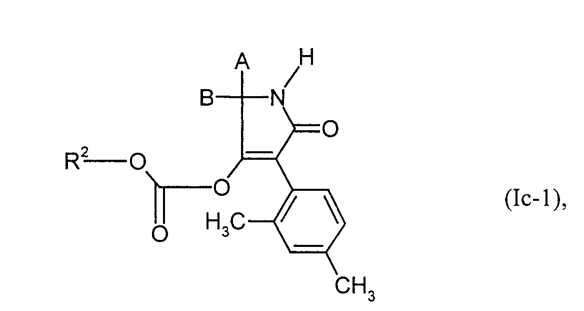 in welcher
A, B und R² die in Anspruch 1 angegebene Bedeutung haben,
man Verbindungen der Formel (I-a) 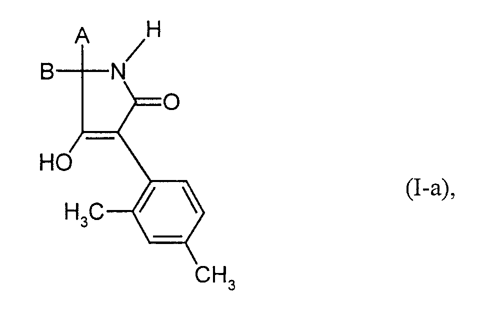 in welcher
A und B die in Anspruch 1 angegebene Bedeutung haben,
mit Verbindungen der allgemeinen Formel (V)
R²-O-CO-Cl (V)
in welcher
R² die in Anspruch 1 angegebene Bedeutung hat,
gegebenenfalls in Gegenart eines Verdünnungsmittels und gegebenenfalls in Gegenwart eines Säurebindemittels umsetzt;
oder
(D) für den Fall von Verbindungen der Formel (Ic-2) 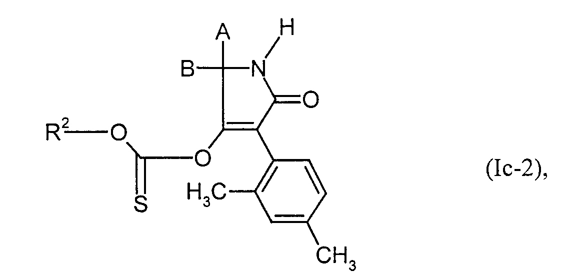 in welcher
A, B und R² die in Anspruch 1 angegebene Bedeutung haben
man Verbindungen der Formel (I-a) 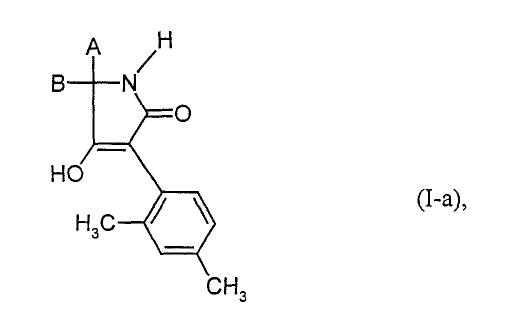 in welcher
A und B die in Anspruch 1 angegebene Bedeutung haben,
α) mit Verbindungen der allgemeinen Formel (VI) 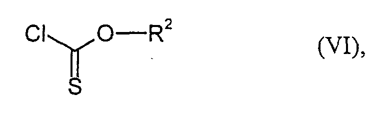 in welcher
R² die in Anspruch 1 angegebene Bedeutung hat,
gegebenenfalls in Gegenwart eines Verdünnungsmittels und gegebenenfalls in Gegenwart eines Säurebindemittels umsetzt.
β) mit Schwefelkohlenstoff und anschließend mit Alkylhalogeniden der allgemeinen Formel (VII)
R²-Hal (VII)
in welcher
R² die oben angegebene Bedeutung hat
und
Hal für Chlor, Brom, Iod steht,
gegebenenfalls in Gegenwart eines Verdünnungsmittels umsetzt.

5. Schädlingsbekämpfungsmittel und Herbizide, gekennzeichnet durch einen Gehalt an mindestens einem Dialkyl-1-H-3-(2,4-dimethylphenyl)-pyrrolidin-2,4-dion der Formel (I-c) gemäß Anspruch 1.

6. Verwendung von Dialkyl-1-H-3-(2,4-dimethylphenyl)-pyrrolidin-2,4-dionen der Formel (I-c) gemäß Anspruch 1 zur Bekämpfung von Schädlingen und unerwünschtem Pflanzenbewuchs.

7. Verfahren zur Bekämpfung von Schädlingen und unerwünschtem Pflanzenbewuchs, dadurch gekennzeichnet, daß man Dialkyl-1-H-3-(2,4-dimethylphenyl)-pyrrolidin-2,4-dione der Formel (I-c) gemäß Anspruch 1 auf Schädlinge, Pflanzen und/oder ihren Lebensraum einwirken läßt.

8. Verfahren zur Herstellung von Schädlingsbekämpfungsmitteln und Herbiziden, dadurch gekennzeichnet, daß man Dialkyl-1-H-3-(2,4-dimethylphenyl)-pyrrolidin-2,4-dione der Formel (I-c) gemäß Anspruch 1 mit Streckmitteln und/oder oberflächenaktiven Mitteln vermischt.

## Claims

1. Dialkyl-1-H-3-(2,4-dimethylphenyl)-pyrrolidine-2,4-diones of the formula (I-c) 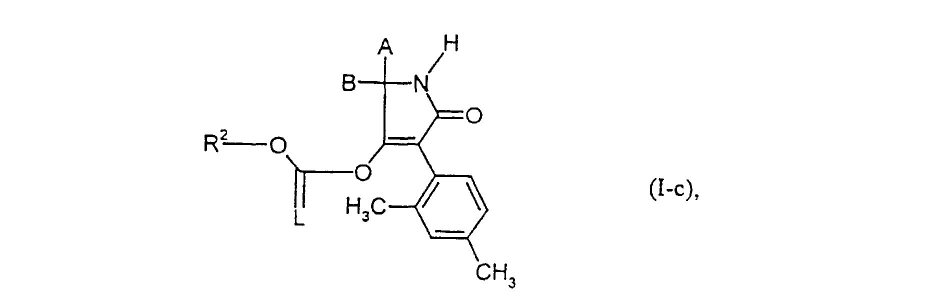 in which
A represents straight-chain or branched C₁-C₁₀-alkyl which is optionally substituted by halogen,
B represents straight-chain or branched C₂-C₁₀-alkyl,
A, B and the carbon atom to which they are bonded preferably represent an unsubstituted C₃-C₁₂-spiro cycle,
L and M represent oxygen or sulphur,
R² represents C₁-C₂₀-alkyl, C₃-C₂₀-alkenyl, C₁-C₈-alkoxy-C₂-C₈-alkyl or C₁-C₈-polyalkoxy-C₂-C₈-alkyl, each of which is optionally substituted by halogen,
or represents cycloalkyl, phenyl or benzyl, each of which is optionally substituted by halogen, nitro, C₁-C₆-alkyl, C₁-C₆-alkoxy or C₁-C₆-halogenoalkyl.

2. Dialkyl-1-H-3-(2,4-dimethylphenyl)-pyrrolidine-2,4-diones of the formula (I-c) according to Claim 1, in which
A represents optionally fluorine- and/or chlorine-substituted straight-chain or branched C₁-C₈-alkyl,
B represents straight-chain or branched C₂-C₈-alkyl
or
A, B and the carbon atom to which they are bonded represent an unsubstituted C₃-C₈-spiro cycle,
L represents oxygen or sulphur,
R² represents C₁-C₁₆-alkyl, C₃-C₁₆-alkenyl, C₁-C₆-alkoxy-C₂-C₆-alkyl or C₁-C₆-polyalkoxy-C₂-C₆-alkyl, each of which is optionally substituted by halogen,
or represents cycloalkyl, phenyl or benzyl, each of which is optionally substituted by halogen, nitro, C₁-C₄-alkyl, C₁-C₃-alkoxy or C₁-C₃-haloalkoxy.

3. Dialkyl-1-H-3-(2,4-dimethylphenyl)-pyrrolidine-2,4-diones of the formula (I-c) according to Claim 1, in which
A represents optionally fluorine-substituted straight-chain or branched C₁-C₆-alkyl,
B represents straight-chain or branched C₂-C₄-alkyl
or
A, B and the carbon atom to which they are bonded represent an unsubstituted C₃-C₇-spiro cycle,
L represents oxygen or sulphur,
R² represents C₁-C₁₄-alkyl, C₃-C₁₄-alkenyl, C₁-C₄-alkoxy-C₂-C₆-alkyl or C₁-C₄-polyalkoxy-C₂-C₆-alkyl, each of which is optionally substituted by fluorine or chlorine,
or represents cycloalkyl, phenyl or benzyl, each of which is optionally substituted by fluorine, chlorine, nitro, methyl, ethyl, propyl, i-propyl, methoxy, ethoxy or trifluoromethyl.

4. Process for the preparation of the dialkyl-1-H-3-(2,4-dimethylphenyl)-pyrrolidine-2,4-diones of the formula (I-c) according to Claim 1, characterized in that
(C) in the case of compounds of the formula (Ic-1) 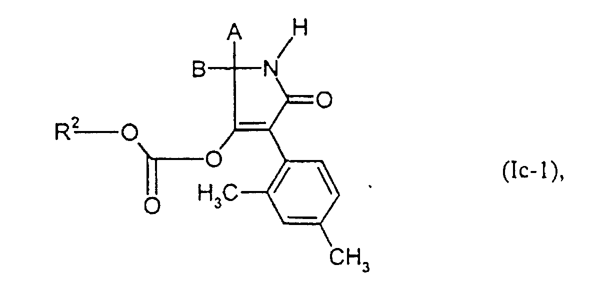 in which
A, B and R² have the meaning given in Claim 1,
compounds of the formula (I-a) 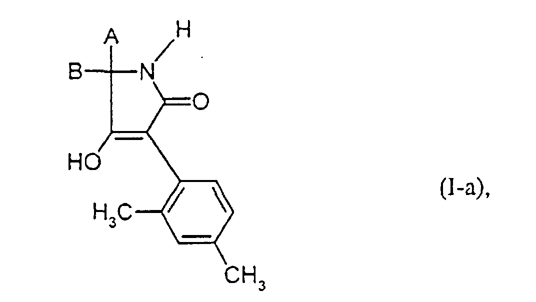 in which
A and B have the meaning given in Claim 1,
are reacted with compounds of the general formula (V)
R²-O-CO-Cl (V)
in which
R² has the meaning given in Claim 1,
if appropriate in the presence of a diluent and if appropriate in the presence of an acid-binding agent;
or
(D) in the case of compounds of the formula (Ic-2) 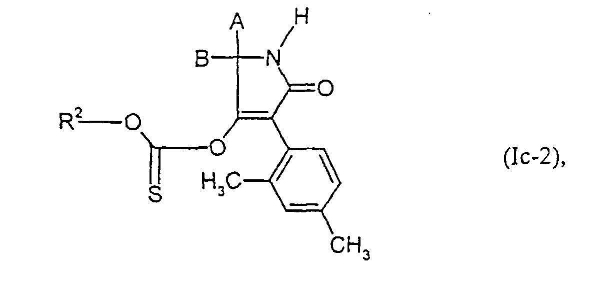 in which
A, B and R² have the meaning given in Claim 1,
compounds of the formula (I-a) 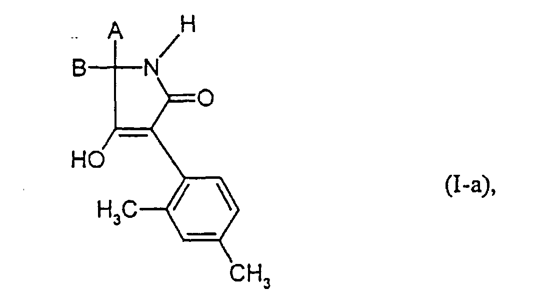 in which
A and B have the meaning given in Claim 1,
are reacted
α) with compounds of the general formula (VI) 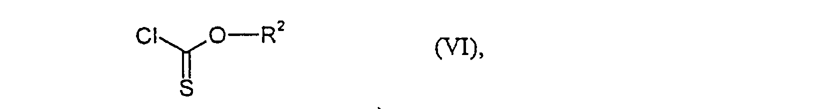 in which
R² has the meaning given in Claim 1,
if appropriate in the presence of a diluent and if appropriate in the presence of an acid-binding agent,
or
β) with carbon disulphide and subsequently with alkyl halides of the general formula (VII)
R²-Hal (VII)
in which
R² has the meaning given above
and
Hal represents chlorine, bromine or iodine,
if appropriate in the presence of a diluent.

5. Pesticides and herbicides, characterized in that they contain at least one dialkyl-1-H-3-(2,4-dimethylphenyl)-pyrrolidine-2,4-dione of the formula (I-c) according to Claim 1.

6. Use of dialkyl-1-H-3-(2,4-dimethylphenyl)-pyrrolidine-2,4-diones of the formula (I-c) according to Claim 1 for combating pests and undesired overgrowth.

7. Method of combating pests and undesired overgrowth, characterized in that dialkyl-1-H-3-(2,4-dimethylphenyl)-pyrrolidine-2,4-diones of the formula (I-c) according to Claim 1 are allowed to act on pests, plants and/or their environment.

8. Process for the preparation of pesticides and herbicides, characterized in that dialkyl-1-H-3-(2,4-dimethylphenyl)-pyrrolidine-2,4-diones of the formula (I-c) according to Claim 1 are mixed with extenders and/or surface-active agents.

## Revendications

1. Dialkyl-1-H-3-(2,4-diméthylphényl)-pyrrolidine-2,4-diones de formule (I-c) 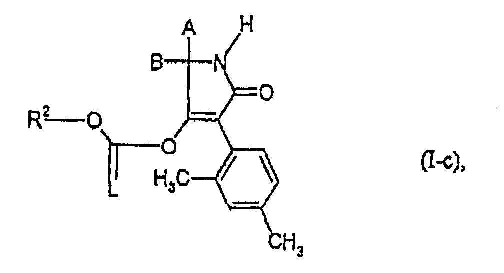 dans laquelle
A représente un groupe alkyle en C₁ à C₁₀ linéaire ou ramifié éventuellement substitué par un halogène,
B représente un groupe alkyle en C₂ à C₁₀ linéaire ou ramifié,
A, B et l'atome de carbone auquel ils sont liés représentent avantageusement un cycle spiranique en C₃ à C₁₂ non substitué,
L représente l'oxygène ou le soufre,
R² est un groupe alkyle en C₁ à C₂₀ éventuellement substitué par un halogène, alcényle en C₃ à C₂₀, (alkoxy en C₁ à C₈)-(alkyle en C₂ à C₈), (polyalkoxy en C₁ à C₈)-(alkyle en C₂ à C₈),
un groupe cycloalkyle, phényle ou benzyle portant éventuellement un substituant halogéno, nitro, alkyle en C₁ à C₆, alkoxy en C₁ à C₆, halogénalkyle en C₁ à C₆.

2. Dialkyl-1-H-3-(2,4-diméthylphényl)-pyrrolidine-2,4-diones de formule (I-c) suivant la revendication 1, dans laquelle
A représente un groupe alkyle en C₁ à C₈ linéaire ou ramifié éventuellement substitué par du fluor et/ou du chlore,
B est un groupe alkyle en C₂ à C₈ linéaire ou ramifié ou bien
A, B et l'atome de carbone auquel ils sont liés représentent un cycle spiranique en C₃ à C₈ non substitué,
L est l'oxygène ou le soufre,
R² est un groupe alkyle en C₁ à C₁₆ éventuellement substitué par un halogène, un groupe alcényle en C₃ à C₁₆, (alkoxy en C₁ à C₆)-(alkyle en C₂ à C₆), (polyalkoxy en C₁ à C₆)-(alkyle en C₂ à C₆),
un groupe cycloalkyle, phényle ou benzyle portant éventuellement un substituant halogéno, nitro, alkyle en C₁ à C₄, alkoxy en C₁ à C₃, halogénalkyle en C₁ à C₃.

3. Dialkyl-1-H-3-(2,4-diméthylphényl)-pyrrolidine-2,4-diones de formule (I-c) suivant la revendication 1, dans laquelle
A est un groupe alkyle en C₁ à C₆ linéaire ou ramifié éventuellement substitué par du fluor,
B est un groupe alkyle en C₂ à C₄ linéaire ou ramifié ou bien
A, B et l'atome de carbone auquel ils sont liés forment un cycle spiranique en C₃ à C₇ non substitué,
L est l'oxygène ou le soufre,
R² représente un groupe alkyle en C₁ à C₁₄ éventuellement substitué par du fluor ou du chlore, un groupe alcényle en C₃ à C₁₄, (alkoxy en C₁ à C₄)-(alkyle en C₂ à C₆), (polyalkoxy en C₁ à C₄)-(alkyle en C₂ à C₆),
un groupe cycloalkyle, phényle ou benzyle portant éventuellement un substituant fluor, chloro, nitro, méthyle, éthyle, propyle, isopropyle, méthoxy, éthoxy, trifluorométhyle.

4. Procédé de production de dialkyl-1-H-3-(2,4-diméthylphényl)-pyrrolidine-2,4-diones de formule (I-c) suivant la revendication 1, caractérisé en ce que,
C) pour le cas de composés de formule (Ic-1) 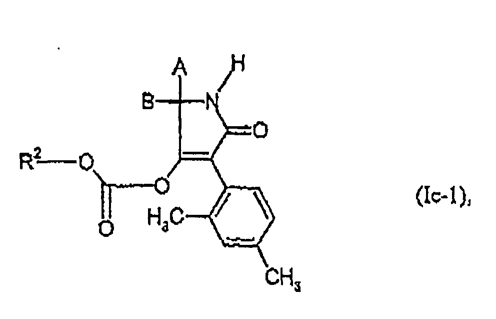 dans laquelle
A, B et R² ont la définition indiquée dans la revendication 1,
on fait réagir des composés de formule (I-a) 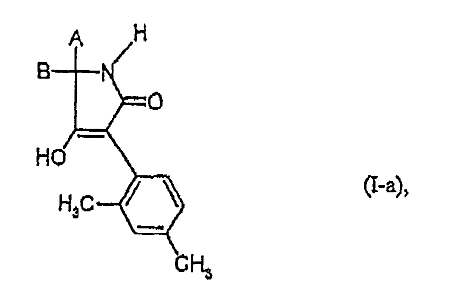 dans laquelle
A et B ont la définition indiquée dans la revendication 1,
avec des composés de formule générale (V)
R²-O-CO-Cl (V)
dans laquelle
R² a la définition indiquée dans la revendication 1, éventuellement en présence d'un diluant et en présence éventuellement d'un accepteur d'acide ;
ou bien
(D) pour le cas de composés de formule (Ic-2) 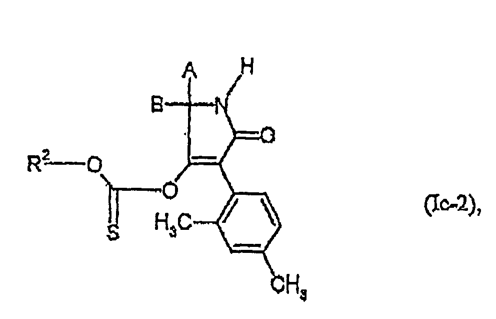 dans laquelle
A, B et R² ont la définition indiquée dans la revendication 1,
on fait réagir des composés de formule (I-a) 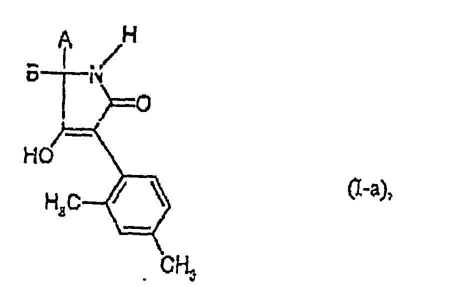 dans laquelle
A et B ont la définition indiquée dans la revendication 1,
α) avec des composés de formule générale (VI) 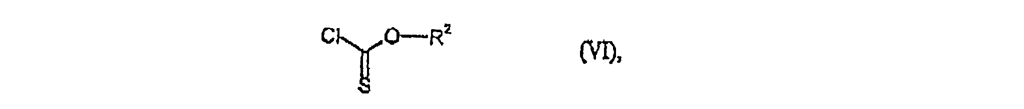 dans laquelle
R² a la définition indiquée dans la revendication 1,
le cas échéant en présence d'un diluant et en présence éventuelle d'un accepteur d'acide,
β) avec du sulfure de carbone, puis avec des halogénures d'alkyle de formule générale (VII)
R²-Hal (VII)
dans laquelle
R² a la définition indiquée ci-dessus
et
Hal représente le chlore, le brome ou l'iode, éventuellement en présence d'un diluant.

5. Pesticides et herbicides, caractérisés par une teneur en au moins une dialkyl-1-H-3-(2,4-diméthylphényl)-pyrrolidine-2,4-dione de formule (I-c) suivant la revendication 1.

6. Utilisation de dialkyl-1-H-3-(2,4-diméthylphényl)-pyrrolidine-2,4-diones de formule (I-c) suivant la revendication 1 pour combattre des parasites et une végétation indésirable.

7. Procédé pour combattre des parasites et une végétation indésirable, caractérisé en ce qu'on fait agir des dialkyl-1-H-3-(2,4-diméthylphényl)-pyrrolidine-2,4-diones de formule (I-c) suivant la revendication 1 sur des parasites, des plantes et/ou leur milieu.

8. Procédé de préparation de pesticides et d'herbicides, caractérisé en ce qu'on mélange des dialkyl-1-H-3-(2,4-diméthylphényl)-pyrrolidine-2,4-diones de formule (I-c) suivant la revendication 1 avec des diluants et/ou des agents tensio-actifs.
